# EUROPEAN PATENT APPLICATION

(11) **EP 2 610 338 A1**
(43) Date of publication of application: **03.07.2013**
(21) Application number: 11814551.5
(22) Date of filing: 28.07.2011
(51) Int. Cl.: C12N 5/10, C12N 5/07, C12P 21/02, C12P 21/08

(54) **METHOD FOR PRODUCING SUBSTANCE**

(30) Priority: 02.08.2010 JP 2010173546
(71) Applicant: Kyowa Hakko Kirin Co., Ltd., Tokyo 100-8185 (JP)
(72) Inventor: OGAWA, Akira, Takasaki-shi Gunma 370-0013 (JP); KURIHASHI, Wataru, Takasaki-shi Gunma 370-0013 (JP); KONNO, Yoshinobu, Takasaki-shi Gunma 370-0013 (JP)
(74) Representative: HOFFMANN EITLE
(86) International application number: PCT/JP2011/067356
(87) International publication number: WO 2012/017925

(57) **Abstract**

The present invention provides a medium suitable for animal cell culture, a culture method using the same, and the like. The present invention relates to a method for culturing animal cells having an ability to produce a substance, which comprises culturing the animal cells in a medium supplemented with an oligopeptide having one or more L-cysteines and excluding glutathione, a method for producing a substance by culturing animal cells having the ability to produce the substance, which comprises culturing the animal cells in a medium supplemented with an oligopeptide having one or more L-cysteines and excluding glutathione to produce and accumulate the substance in the culture, and collecting the substance from the culture, and a culture medium comprising an oligopeptide having one or more L-cysteines and excluding glutathione.

## Description

### TECHNICAL FIELD

The present invention relates to a method for culturing animal cells having an ability to produce a substance, which comprises culturing the animal cells in a medium supplemented with an oligopeptide having one or more L-cysteines and excluding glutathione, a method for producing the substance by culturing animal cells having the ability to produce the substance, which comprises culturing the animal cells in a medium supplemented with an oligopeptide having one or more L-cysteines and excluding glutathione to produce and accumulate the substance in the culture, and collecting the substance from the culture, and a culture medium comprising an oligopeptide having one or more L-cysteines and excluding glutathione.

### BACKGROUND ART

Glycoproteins, in particular, antibodies have been recently approved as medicines, and more candidate antibodies are currently under development (Non-Patent Literature 1). It is expected that the use of animal cells for the production of substances such as antibodies and the like will expand in the future. However, it has been pointed out that sufficient substance productivity by the use of animal cells has not been yet achieved.

Animal cells such as Chinese hamster ovary cell (CHO cell), hybridoma or the like are selected as host cells for the production of glycoproteins including antibodies, because sugar chains bound to glycoproteins produced in the above cells are relatively similar to that in human cells (Patent Literatures 1, 2, Non-Patent Literature 2).

Although some efforts for medium improvement have been made for the purpose of increasing substance productivity (Patent Literatures 5, 6), cysteine, cystine or a salt thereof have problems such as precipitation and the like in the medium due to low solubility of cystine [0.1 g/L (H₂O, 20°C)] (Non-Patent Literature 2) and low stability of cysteine hydrochloride in an aqueous solution (Non-Patent Literature 3), and thus there are some limitations in industrial applications. On the other hand, with respect to the use of short-chain peptides such as dipeptides and the like in cell culture, a basal medium supplemented with L-amino acid-L-glutamine for the purpose of heat sterilization of the medium is known (Patent Literatures 3, 4).

Intravenous, oral and parenteral administrations of cysteine-containing oligopeptides: N,N'-bis-L-alanyl-L-cystine, N,N'-bis-L-glycyl-L-cystine, N,N'-bis-L-leucyl-L-cystine, and N,N'-bis-L-isoleucyl-L-cystine are also known (Patent Literature 7, Non-Patent Literatures 3, 4). Glutathione, a cysteine-containing tripeptide, is composed of glutamic acid, cysteine or glycine, and its addition to the medium is known (Patent Literature 8).

However, applications of other L-cysteine-containing oligopeptides to the media have not been known.

### CITATION LIST

### PATENT LITERATURES

Patent Literature 1: International Publication WO 1996/39488
Patent Literature 2: US Patent No. 4724206
Patent Literature 3: JP Patent Publication No. 61-271985
Patent Literature 4: EP Patent No. 0220379
Patent Literature 5: International Publication WO 2008/136398
Patent Literature 6: International Publication WO 2008/141207
Patent Literature 7: International Publication WO 1988/03150
Patent Literature 8: JP Patent Publication No. 2002-142759

### NON-PATENT LITERATURES

Non-Patent Literature 1: Nature Rev. Drug. Discov., 3, 383(2004)
Non-Patent Literature 2: Nat Biotechnol., 22, 1393-8(2004)
Non-Patent Literature 3: J. Nutri., 131, 2562s-2568s, (2001)
Non-Patent Literature 4: Zeitschrift fuer Ernaehrungswissenschaft, 28, 191-200(1989)

### SUMMARY OF THE INVENTION

### PROBLEMS TO BE SOLVED BY THE INVENTION

With regard to a method for producing a substance from animal cells by culturing the cells, there is a need to develop a method for improving the substance productivity and a medium that is applicable to the industry for the improvement of the substance productivity.

### MEANS FOR SOLVING THE PROBLEMS

That is, the present invention is as follows.
1. A method for culturing an animal cell having an ability to produce a substance, which comprises culturing the animal cell in a medium supplemented with an oligopeptide having one or more L-cysteines and excluding glutathione.
2. The method described in 1 above, wherein the oligopeptide having one or more L-cysteines is a dipeptide or a dimer of the dipeptide.
3. The method described in 2 above, wherein the dipeptide having one or more L-cysteines is glycyl-L-cysteine, L-alanyl-L-cysteine, or γ-glutamyl cysteine, or a salt thereof.
4. The method described in 2 above, wherein the dimer of the dipeptide having one or more L-cysteines is bis (glycyl-L-cysteine) or bis (L-alanyl-L-cysteine).
5. The method described in any one of 1 to 4 above, wherein the animal cell is cultured in a medium further supplemented with L-tyrosine or an oligopeptide having one or more L-tyrosines.
6. The method described in 5 above, wherein the oligopeptide having one or more L-tyrosines is a dipeptide.
7. The method described in 6 above, wherein the dipeptide having one or more L-tyrosines is L-alanyl-L-tyrosine or a salt thereof.
8. The method described in any one of 1 to 7 above, wherein the oligopeptide having one or more L-cysteines which is added to the medium has a concentration of from 0.01 to 100 mmol/L.
9. The method described in any one of 5 to 8 above, wherein L-tyrosine or the oligopeptide having one or more L-tyrosines which is added to the medium has a concentration of from 0.01 to 100 mmol/L.
10. The method described in any one of 1 to 9 above, wherein the animal cell is derived from an animal belonging to mammals.
11. The method described in 10 above, wherein the animal belonging to mammals belongs to primates or rodents.
12. The method described in 10 or 11 above, wherein the animal cell is a myeloma cell or an ovarian cell, or a cell derived therefrom.
13. The method described in any one of 1 to 12 above, wherein the substance is a peptide.
14. The method described in any one of 10 to 12 above, wherein the animal cell is a transformed cell to which a vector comprising a gene encoding the peptide is introduced.
15. The method described in 13 or 14 above, wherein the peptide is a glycoprotein.
16. The method described in 15 above, wherein the glycoprotein is an antibody.
17. The method described in any one of 1 to 16 above, wherein the oligopeptide is added at logarithmic growth phase of the cell.
18. The method described in any one of 1 to 17 above, wherein the medium supplemented with an oligopeptide is a feed medium.
19. The method described in any one of 1 to 18 above, wherein the culture method is a culture method using a culture tank.
20. A method for producing a substance by culturing an animal cell having an ability to produce a substance, which comprises culturing the animal cell in a medium supplemented with an oligopeptide having one or more L-cysteines and excluding glutathione to produce and accumulate the substance in the culture, and collecting the substance from the culture.
21. The method described in claim 20, wherein the oligopeptide having one or more L-cysteines is a dipeptide or a dimer of the dipeptide.
22. The method described in 21 above, wherein the dipeptide having one or more L-cysteines is glycyl-L-cysteine, L-alanyl-L-cysteine, or γ-glutamyl cysteine, or a salt thereof.
23. The method described in 21 above, wherein the dimer of the dipeptide having one or more L-cysteines is bis (glycyl-L-cysteine) or bis (L-alanyl-L-cysteine).
24. The method described in any one of 20 to 23 above, wherein the animal cell is cultured in a medium further supplemented with L-tyrosine or an oligopeptide having one or more L-tyrosines.
25. The method described in 24 above, wherein the oligopeptide having one or more L-tyrosines is a dipeptide.
26. The method described in 25 above, wherein the dipeptide having one or more L-tyrosines is L-alanyl-L-tyrosine or a salt thereof.
27. The method described in any one of 20 to 26 above, wherein the oligopeptide having one or more L-cysteines which is added to the medium has a concentration of from 0.01 to 100 mmol/L.
28. The method described in any one of 24 to 27 above, wherein L-tyrosine or the oligopeptide having one or more L-tyrosines which is added to the medium has a concentration of from 0.01 to 100 mmol/L.
29. The method described in any one of 20 to 28 above, wherein the animal cell is derived from an animal belonging to mammals.
30. The method described in 29 above, wherein the animal belonging to mammals belongs primates or rodents.
31. The method described in 29 or 30 above, wherein the animal cell is a myeloma cell or an ovarian cell, or a cell derived therefrom.
32. The method described in any one of 20 to 31 above, wherein the substance is a peptide.
33. The method described in any one of 29 to 31 above, wherein the animal cell is a transformed cell to which a vector comprising a gene encoding the peptide is introduced.
34. The method described in 32 or 33 above, wherein the peptide is a glycoprotein.
35. The method described in 34 above, wherein the glycoprotein is an antibody.
36. The method described in any one of 20 to 35 above, wherein the oligopeptide is added at logarithmic growth phase of the cell.
37. The method described in any one of 20 to 36 above, wherein the medium supplemented with the oligopeptide is a feed medium.
38. The method described in any one of 20 to 37 above, wherein the culture method is a culture method using a culture tank.
39. A medium comprising an oligopeptide having one or more L-cysteine and excluding glutathione.
40. The medium described in 39 above, wherein the oligopeptide having one or more L-cysteines and excluding glutathione is a dipeptide or a dimer of the dipeptide.
41. The medium described in 40 above, wherein the dipeptide having one or more L-cysteines is glycyl-L-cysteine, L-alanyl-L-cysteine, or γ-glutamyl cysteine, or a salt thereof.
42. The medium described in 41 above, wherein the dimer of the dipeptide having one or more L-cysteines is bis (glycyl-L-cysteine) or bis (L-alanyl-L-cysteine).
43. The medium described in any one of 39 to 42 above, wherein the medium further comprises L-tyrosine or an oligopeptide having one or more L-tyrosines.
44. The medium described in 43 above, wherein the oligopeptide having one or more L-tyrosines is a dipeptide.
45. The medium described in 44 above, wherein the dipeptide having one or more L-tyrosines is L-alanyl-L-tyrosine or a salt thereof.
46. The medium described in any one of 39 to 45 above, wherein the oligopeptide having one or more L-cysteines has a concentration of from 0.01 to 100 mmol/L.
47. The medium described in any one of 39 to 45 above, wherein a L-tyrosine or the oligopeptide having one or more L-tyrosines has a concentration of from 0.01 to 100 mmol/L.

### EFFECT OF THE INVENTION

The present invention relates to a method for culturing animal cells having an ability to produce a substance, which comprises culturing the animal cells in a medium supplemented with an oligopeptide having one or more L-cysteines and excluding glutathione, a method for producing a substance by culturing animal cells having the ability to produce the substance, which comprises culturing the animal cells in a medium supplemented with an oligopeptide having one or more L-cysteines and excluding glutathione to produce and accumulate the substance in the culture, and collecting the substance from the culture, and a culture medium including an oligopeptide having one or more L-cysteines and excluding glutathione.

### EMBODIMENTS FOR CARRYING OUT THE INVENTION

The present invention relates to a method for culturing animal cells having an ability to produce a substance, which comprises culturing the animal cells in a medium supplemented with an oligopeptide having one or more L-cysteines and excluding glutathione.

The oligopeptide used in the present invention is not particularly limited, but preferably a peptide composed of from 2 to 20 amino acids, more preferably a peptide composed of from 2 to 10 amino acids, for example, a pentapeptide, a tetrapeptide, a tripeptide, a dipeptide or a dimer of these peptides. The oligopeptide used in the present invention may be also in a form of a salt such as hydrochloride salts, sodium salts or the like, and/or in a form of a solvate such as hydrates or the like.

Specific examples of the dipeptides having one or more L-cysteines may include glycyl-L-cysteine (Gly-Cys), L-alanyl-L-cysteine (Ala-Cys), L-serinyl-L-cysteine (Ser-Cys), L-cysteinyl-L-cysteine (Cys-Cys), L-lysinyl-L-cysteine (Lys-Cys), L-methionyl-L-cysteine (Met-Cys), L-phenylalanyl-L-cysteine (Phe-Cys), L-tyrosinyl-L-cysteine (Tyr-Cys), L-tryptophanyl-L-cysteine (Trp-Cys), L-glutamyl-L-cysteine (Glu-Cys), L-asparaginyl-L-cysteine (Asp-Cys), L-glutaminyl-L-cysteine (Gln-Cys), L-histidyl-L-cysteine (His-Cys), L-arginyl-L-cysteine (Arg-Cys), L-cysteinyl-glycine (Cys-Gly), L-cysteinyl-L-alanine (Cys-Ala), L-cysteinyl-L-serine (Cys-Ser), L-cysteinyl-L-tyrosine (Cys-Tyr), L-cysteinyl-L-leucine (Cys-Leu), L-cysteinyl-L-isoleucine (Cys-Ile), L-cysteinyl-L-methionine (Cys-Met), L-cysteinyl-L-phenylalanine (Cys-Phe), L-cysteinyl-L-tryptophan (Cys-Trp), L-cysteinyl-L-histidine (Cys-His), L-cysteinyl-L-arginine (Cys-Arg) and the like (International Publication WO 2004/058960). Further, specific examples of the dimer of the dipeptides having one or more L-cysteines may include bis (glycyl-L-cysteine), bis (L-alanyl-L-cysteine) and the like.

The present invention relates to a medium comprising an oligopeptide having one or more L-cysteines and excluding glutathione.

Further, the medium of the present invention may be further supplemented with L-tyrosine or an oligopeptide having one or more L-tyrosines. L-tyrosine added to the medium of the present invention may be in a form of a salt such as hydrochloride salts, sodium salts or the like, and/or in a form of a solvate such as hydrates or the like.

The medium including an oligopeptide is not particularly limited, but the oligopeptide is preferably added to at least one medium selected from a medium for expansion culture, a basal (initial) medium, a perfusion medium, or a feed medium.

The oligopeptide having one or more L-cysteines of the present invention may be prepared by the known synthetic method, enzymatic method or fermentation method. Examples of the preparation method of L-glycyl-L-cysteine, L-alanyl-L-cysteine, bis (L-glycyl-L-cysteine), or bis (L-alanyl-L-cysteine) may include the synthetic method, the fermentation method (International Publication WO 2004/058960, International Publication WO 2006/001379) or the like.

Further, in the method for culturing animal cells of the present invention, L-tyrosine or an oligopeptide having one or more L-tyrosine may be further added.

Among the oligopeptides having one or more L-tyrosines, specific examples of the dipeptide having one or more L-tyrosines may include glycyl-L-tyrosine (Gly-Tyr), L-alanyl-L-tyrosine (Ala-Tyr), L-serinyl-L-tyrosine (Ser-Tyr), L-valinyl-L-tyrosine (Val-Tyr), L-methionyl-L-tyrosine (Met-Tyr), L-phenylalanyl-L-tyrosine (Phe-Tyr), L-arginyl-L-tyrosine (Arg-Tyr), L-tyrosinyl-glycine (Tyr-Gly), L-tyrosinyl-L-alanine (Tyr-Ala), L-tyrosinyl-L-serine (Tyr-Ser), L-tyrosinyl-L-methionine (Tyr-Met), L-tyrosinyl-L-arginine (Tyr-Arg) and the like.

The oligopeptide having one or more L-tyrosines may be also prepared by the known synthetic method, enzymatic method or fermentation method. Examples of the preparation method of L-alanyl-L-tyrosine may include the synthetic method, the fermentation method (International Publication WO 2004/058960, International Publication WO 2006/001379) or the like.

The method of adding the oligopeptide to the medium is not particularly limited, but the oligopeptide is preferably added to at least one medium selected from the expansion culture medium, the basal (initial) medium, the perfusion medium, or the feed medium.

In the present invention, the feed medium means a medium added aside from the basal medium. Further, the oligopeptide having one or more L-cysteines, and L-tyrosine or oligopeptide having one or more L-tyrosines may be added to one medium at the same time, or separately added to two or more media.

When added as the feed medium, the oligopeptide may be fed to the medium alone as the feed medium, or a premixture of the oligopeptide and other medium ingredients may be fed to the medium as the feed medium. Further, the feed medium may be separately and discontinuously fed to the medium, or continuously fed to the medium.

A preservation method of the feed medium is not particularly limited, as long as it is able to maintain the medium in an aseptic condition, and examples thereof may include a method using a stainless steel tank, a disposable bag or the like.

A concentration of the oligopeptide having one or more L-cysteines that is added to each media may be properly selected depending on the type of animal cells used in the culture, the type of the substance to be produced, the type of the oligopeptide, the addition timing of the oligopeptide and the like, and is preferably 0.001 to 1000 mmol/L, more preferably 0.005 to 500 mmol/L, and particularly preferably 0.01 to 100 mmol/L.

A concentration of L-tyrosine or the oligopeptide having one or more L-tyrosines that is added to the media may be also properly selected depending on the type of animal cells used in the culture, the type of the substance to be produced, the type of the oligopeptide, the addition timing of the oligopeptide and the like, and is preferably 0.001 to 1000 mmol/L, more preferably 0.005 to 500 mmol/L, and particularly preferably 0.01 to 100 mmol/L.

The timing of adding the feed medium including the oligopeptide to the basal medium is not particularly limited. It can be added before/after the initiation of cultivation, but it is preferably added to the medium at logarithmic growth phase. The logarithmic growth phase varies depending on a cell line being cultured and a culture control method, but refers to a period in which the number of cells in the culture increases until the number of cells in the culture becomes constant.

The feed medium including the oligopeptide may be added to the basal medium at any mixing ratio, but preferably at the mixing ratio from 10% to 50%, and more preferably from 20% to 30% due to restrictions on industrial equipment.

Any medium may be used as the medium used in the present invention, as long as it can be used in the culture of animal cells. The media used generally in the culture of animal cells are used. Cysteine may be included as the medium component in advance, or not included.

Examples of the medium used in the present invention may include a basal medium, a serum-containing medium, a serum-free medium, a medium containing no animal-derived ingredients, a protein-free medium, a complete synthetic medium and the like, and the serum-free medium, the protein-free medium, or the complete synthetic medium is preferred.

Examples of the basal medium may include an RPMI1640 medium [The Journal of the American Medical Association, 199, 519(1967)], an Eagle's MEM medium [Science, 130, 432(1959)], a Dulbecco's Modified MEM (DMEM) medium [Virology, 8, 396(1959)], an 199 medium [Proceeding of the Society for the Biological Medicine, 73, 1(1950)], an F12 medium (manufactured by LTI) [Proc. Natl. Acad. Sci. USA, 53, 288(1965)], an Iscove's Modified Dulbecco's Medium (IMDM medium) [J. Experimental Medicine, 147, 923(1978)], an EX-CELL (registered trademark) 302 medium (manufactured by SAFC bioscience) or modified media thereof, or mixtures thereof, or the like.

Of them, the RPMI1640 medium, the DMEM medium, the F 12 medium, the IMDM and the EX-CELL (registered trademark) 302 medium, the EX-CELL (registered trademark) 325 PF medium (manufactured by SAFC bioscience), the EX-CELL (registered trademark) CD CHO medium (manufactured by SAFC bioscience) or the like are preferred.

Examples of the serum-free medium may include media that are prepared by adding to the basal medium, physiologically active substances as alternatives to serum, nutritional factors, carbon or nitrogen sources assimilated by animal cells or the like. These additives are preferably added to the medium prior to the culture.

Examples of the nutritional factors may include glucose, amino acids, vitamins and the like.

Examples of the amino acids may include L-alanine(Ala), L-arginine (Arg), L-asparagine (Asn), L-aspartic acid (Asp), L-cysteine (Cys), L-cystine, L-glutamic acid (Glu), L-glutamine (Gln), glycine (Gly), L-histidine (His), L-isoleucine (Ile), L-leucine (Leu), L-lysine (Lys), L-methionine (Met), L-phenylalanine (Phe), L-proline (Pro), L-serine (Ser), L-threonine (Thr), L-tryptophan (Trp), L-valine (Val) and the like, and they are used alone or in combinations of two or more thereof. Further, salts such as hydrochloride salts thereof and sodium salts thereof, and/or solvates such as hydrates thereof may be used.

Examples of the vitamins may include d-biotin, D-pantothenic acid, choline, folate, myo-inositol, niacinamide, pyridoxal, riboflavin, thiamine, cyanocobalamine, DL-α-tocopherol and the like, and they are used alone or in combinations of two or more thereof. Further, salts such as hydrochloride salts thereof and sodium salts thereof, and/or solvates such as hydrates thereof may be used.

Examples of the physiologically active substances may include insulin, IGF-I, transferrin, serum albumin, a serum fraction containing growth factors and the like.

In the medium containing no animal-derived ingredients, examples of substances added instead of animal-derived ingredients may include physiologically active substances prepared by genetic recombination, hydrolysates, lipids containing no animal-derived raw materials and the like.

Examples of the hydrolysates may include hydrolysates of soybean, wheat, rice, pea, cotton seed, yeast extract or the like.

Examples of the lipids may include cholesterol, linoleic acid, linolenic acid and the like. Further, salts such as hydrochloride salts thereof and sodium salts thereof and/or solvates such as hydrates thereof may be used.

Examples of the protein-free medium may include an ADPF medium (Animal derived protein free medium, manufactured by HyClone), an EX-CELL (registered trademark) 325 PF medium (manufactured by SAFC bioscience) and the like.

Examples of the complete synthetic medium may include a CD CHO Fusion medium (manufactured by SAFC bioscience), a CD-Hybridoma medium (manufactured by Invitrogen), a CD-CHO medium (manufactured by Invitrogen), an IS-CD-CHO medium (manufactured by Irvine scientific), an EX-CELL (registered trademark) CD CHO medium (manufactured by SAFC bioscience) and the like.

If a long-term or high density culture is carried out, a medium containing high concentrations of amino acids and vitamins, for example, a medium containing RPMI1640 medium, DMEM medium and F12 medium at a ratio of 1:1:1, a medium containing DMEM medium and F12 medium at a ratio of 1:1, a hybridoma SFM medium (manufactured by Invitrogen) or the like may be used.

Examples of the animal cells having the ability to produce the substance used in the present invention may include cells derived from mammals, the bird, the reptile, the amphibian, the fish, the insect and the like. Among them, cells of animal belonging to the mammalian are preferred, and animal cells derived from primates such as human, monkey and the like, or animal cells derived from rodents such as mouse, rat, hamster and the like are more preferred.

The cells belonging to mammals are preferably myeloma cells, ovarian cells, renal cells, blood cells, uterine cells, connective tissue cells, mammary cells, embryonic retinoblastoma cells, or cells derived therefrom, and more preferably cells selected from myeloma cells, myeloma cell-derived cells, ovarian cells, and ovarian cell-derived cells.

Examples of the cells belonging to mammals may include human cell lines such as HL-60 (ATCC No. CCL-240), HT-1080 (ATCC No. CCL-121), HeLa (ATCC No. CCL-2), 293 (ECACC No. 85120602), Namalwa (ATCC CRL-1432), Namalwa KJM-1 [Cytotechnology, 1, 151(1988)], NM-F9 (DSM ACC2605, International Publication WO 2005/017130) and PER.C6 (ECACC No. 96022940, US Patent No. 6855544), monkey cell lines such as VERO (ATCC No. CCL-1651) and COS-7 (ATCC No. CRL-1651), mouse cell lines such as C127I (ATCC No. CRL-1616), Sp2/0-Ag14 (ATCC No. CRL-1581), and NIH3T3 (ATCC No. CRL-1658), NS0 (ATCC No. CRL-1827), rat cell lines such as Y3 Ag 1.2.3. (ATCC No. CRL-1631), YO (ECACC No. 85110501) and YB2/0 (ATCC No. CRL-1662), hamster cell lines such as CHO-K1 (ATCC No. CCL-61), CHO/dhfr- (ATCC No. CRL-9096), CHO/DG44 [Proc. Natl. Acad. Sci. USA, 77, 4216 (1980)] and BHK21 (ATCC No. CRL-10), dog cell lines such as MDCK (ATCC No. CCL-34), and the like.

Examples of the cells belonging to the bird may include a chicken cell line SL-29 (ATCC No. CRL-29) and the like. Examples of the cells belonging to the fish may include a zebra fish cell line ZF4 (ATCC No. CRL-2050) and the like.

Examples of the cells belonging to the insect may include a moth (Spodoptera frugiperda) cell line Sf9 (ATCC No. CRL-1711) and the like. Examples of the primary culture cells used in the vaccine preparation may include primary monkey kidney cells, primary rabbit kidney cells, primary chicken embryonic cells, primary quail embryonic cells and the like.

Examples of the myeloma cell or myeloma cell-derived cells may include Sp2/0-Ag14, NS0, Y3 Ag 1.2.3., YO or YB2/0 and the like. Examples of the ovarian cells or ovarian cell-derived cells may include CHO-K1, CHO/dhfr-, CHO/DG44 and the like.

Further, Examples of the renal cells may include 293, VERO, COS-7, BHK21, MDCK and the like. Examples of the blood cells may include HL-60, Namalwa, Namalwa KJM-1, NM-F9 and the like.

Examples of the uterine cells may include HeLa and the like. Examples of the connective tissue cells may include HT-1080, NIH3T3 and the like. Examples of the mammary cells may include C1271I and the like. Examples of the embryonic retinoblastoma cells may include PER.C6 and the like.

Examples of the animal cells having the ability to produce the substance used in the present invention may include fusion cells prepared to produce the substance or the like, in addition to the animal cells producing the substance. For example, if the desired substance is an antibody, examples of the cells may include hybridoma cells that are formed from the fusion of antibody-producing cells such as B-cells or the like with myeloma cells.

Further, animal cells that are mutated to produce the substance, animal cells that are mutated to have an increased expression level of the substance or the like are also included in the animal cells of the present invention.

Examples of the animal cells that are mutated to produce the substance may include cells in which mutations are introduced in protein modification enzymes in order to produce the desired substance or the like. For example, if the desired substance is a glycoprotein, examples of the cells may include cells in which mutations are introduced in a variety of sugar chain modification enzymes in order to change the structure of sugar chains or the like.

Further, the animal cells producing the substance of the present invention also include, for example, animal cells that are transformed with a recombinant vector including a gene involved in the production of the substance. The transformed cells may be obtained by introduction of the recombinant vector including DNA involved in the production of the substance and a promoter into the animal cells having the ability to produce the substance.

Examples of the DNA involved in the production of the substance may include DNA encoding a substance such as peptides or the like, and DNA encoding an enzyme or a protein that is involved in the biosynthesis of the substance and the like.

Any substance may be used as the substance produced by the method of the present invention, as long as it can be produced by animal cells, and a substance that can be produced by cells of animal belonging to the mammalian is preferred. Examples thereof may include biocatalytic molecules such as peptides, ribozymes or the like, structure formation/maintenance molecules such as keratin, collagen, elastin, resilin, fibroin or the like, vaccines such as variolo vaccine, polio vaccine, measles vaccine, rubella vaccine, mumps vaccine, rabies vaccine, varicella vaccine, bovine ephemeral fever vaccine, ibaraki disease vaccine, bovine infectious tracheitis vaccine or the like, viruses such as adenovirus, baculovirus or the like.

The peptide is preferably peptides derived from eukaryotic cells, more preferably peptides derived from animal cells, and examples thereof may include peptides derived from mammalian cells. Further, the peptide of the present invention may be in any form, as long as it includes the desired peptide and has the activity, and the peptide may be, for example, the artificially modified peptides such as fusion peptides prepared by fusion with other peptides and the like, or peptides composed of partial peptide fragments.

Specific examples of the peptide may include glycoproteins, peptides having a physiological activity and the like.

Specific examples of the glycoprotein may include an antibody, erythropoietin (EPO) [J. Biol. Chem., 252, 5558 (1977)], thrombopoietin (TPO) [Nature, 369 533 (1994)], a tissue-type plasminogen activator, pro-urokinase, thrombomodulin, antithrombin III, protein C, protein S, blood coagulation factor VII, blood coagulation factor VIII, blood coagulation factor IX, blood coagulation factor X, blood coagulation factor XI, blood coagulation factor XII, a prothrombin complex, fibrinogen, albumin, gonadotropic hormone, thyroid-stimulating hormone, epidermal growth factor (EGF), hepatocyte growth factor (HGF), keratinocyte growth factor, activin, osteogenic factor, stem cell factor (SCF), granulocyte colony stimulating factor (G-CSF) [J. Biol. Chem., 258, 9017 (1983)], macrophage colony stimulating factor (M-CSF) [J. Exp. Med., 173, 269 (1991)], granulocyte-macrophage colony stimulating factor (GM-CSF) [J. Biol. Chem., 252, 1998 (1977)], interferon α, interferon β, interferon γ, interleukin-2 (IL-2) [Science, 193, 1007 (1976)], interleukin 6, interleukin 10, interleukin 11, interleukin-12 (IL-12) [J. Leuk. Biol., 55, 280 (1994)], soluble interleukin 4 receptor, tumor necrosis factor α, DNaseI, galactosidase, α glucosidase, glucocerebrosidase, hemoglobin or transferrin, derivatives thereof, partial glycoprotein fragments thereof and the like.

Any antibody may be used, as long as it has an antigen-binding activity, and examples thereof may include antibodies recognizing tumor-associated antigens or antibody fragments thereof, antibodies recognizing allergy or inflammation-associated antigens or antibody fragments thereof, antibodies recognizing cardiovascular disease-associated antigens or antibody fragments thereof, antibodies recognizing autoimmune disease-associated antigens or antibody fragments thereof, antibodies recognizing viral or bacterial infection-associated antigens or antibody fragments thereof and the like.

Examples of tumor-associated antigens may include CD1a, CD2, CD3, CD4, CD5, CD6, CD7, CD9, CD10, CD13, CD19, CD20, CD21, CD22, CD25, CD28, CD30, CD32, CD33, CD38, CD40, CD40 ligand (CD40L), CD44, CD45, CD46, CD47, CD52, CD54, CD55, CD56, CD59, CD63, CD64, CD66b, CD69, CD70, CD74, CD80, CD89, CD95, CD98, CD105, CD134, CD137, CD138, CD147, CD158, CD160, CD162, CD164, CD200, CD227, adrenomedullin, angiopoietin related protein 4 (ARP4), aurora, B7-H1, B7-DC, integlin, bone marrow stromal antigen 2 (BST2), CA125, CA19.9, carbonic anhydrase 9 (CA9), cadherin, cc-chemokine receptor (CCR) 4, CCR7, carcinoembryonic antigen (CEA), cysteine-rich fibroblastgrowth factor receptor-1 (CFR-1), c-Met, c-Myc, collagen, CTA, connective tissuegrowth factor (CTGF), CTLA-4, cytokeratin-18, DF3, E-catherin, epidermalgrowth facter receptor (EGFR), EGFRvIII, EGFR2 (HER2), EGFR3 (HER3), EGFR4 (HER4), endoglin, epithelial cell adhesion molecule (EpCAM), endothelial protein C receptor (EPCR), ephrin, ephrin receptor (Eph), EphA2, endotheliase-2 (ET2), FAM3D, fibroblast activating protein (FAP), Fc receptor homolog 1 (FcRH1), ferritin, fibroblastgrowth factor8 (FGF8), FGF8 receptor, basic FGF (bFGF), bFGF receptor, FGF receptor (FGFR) 3, FGFR4, FET1, FLT3, folate receptor, frizzled homologue 10 (FZD 10), frizzled receptor 4 (FZD-4), G250, G-CSF receptor, ganglioside (for example, GD2, GD3, GM2, GM3 or the like), globo H, gp75, gp88, GPR-9-6, heparanase I, hepatocytegrowth factor (HGF), HGF receptor, HLA antigen (for example, HLA-DR or the like), HM1.24, human milk fatglobule (HMFG), hRS7, heat shock protein 90 (hsp90), idiotype epitope, insulin-likegrowth factor (IGF), IGF receptor (IGFR), interleukin (for example, IL-6, IL-15 or the like), interleukin receptor (for example, IL-6R, IL-15R or the like), integrin, immune receptor translocation associated-4 (IRTA-4), kallikrein 1, KDR, KIR2DL1, KIR2DL2/3, KS1/4, lamp-1, lamp-2, laminin-5, Lewis y, sialyl Lewis x, lymphotoxin-beta receptor (LTBR), LUNX, melanoma-associated chondroitin sulfate proteoglycan (MCSP), mesothelin, MICA, Mullerian inhibiting substance type II receptor (MISIIR), mucin, neural cell adhesion molecule (NCAM), Necl-5, Notch1, osteopontin, platelet-derivedgrowth factor (PDGF), PDGF receptor, platelet factor-4 (PF-4), phosphatidylserine, Prostate Specific Antigen (PSA), prostate stem cell antigen (PSCA), prostate specific membrane antigen (PSMA), Parathyroid hormone related protein/peptide (PTHrP), receptor activator of NF-kappaB ligand (RANKL), receptor for hyaluronic acid mediated motility (RHAMM), ROBO1, SART3, semaphorin 4B (SEMA4B), secretory leukocyte protease inhibitor (SLPI), SM5-1, sphingosine-1-phosphate, tumor-associatedglycoprotein-72 (TAG-72), transferrin receptor (TfR), TGF-beta, Thy-1, Tie-1, Tie2 receptor, T cell immunoglobulin domain and mucin domain 1 (TIM-1), human tissue factor (hTF), Tn antigen, tumor necrosis factor (TNF), Thomsen-Friedenreich antigen (TF antigen), TNF receptor, tumor necrosis factor-related apoptosis-inducing ligand (TRAIL), TRAIL receptor (for example, DR4, DR5 or the like), system ASC amino acid transporter 2 (ASCT2), trkC, TROP-2, TWEAK receptor Fn14, type IV collagenase, urokinase receptor, vascular endothelialgrowth factor (VEGF), VEGF receptor (for example, VEGFR1, VEGFR2, VEGFR3 or the like), vimentin, VLA-4, and the like.

The antibody may be any one of monoclonal antibody or polyclonal antibody, and examples of the antibody class may include immunoglobulin G (IgG), immunoglobulin A (IgA), immunoglobulin E (IgE) and immunoglobulin M (IgM). Among these, IgG is preferred. Further, examples of the IgG subclass may include IgG1, IgG2, IgG3 or IgG4.

The antibody may also include a fragment including a part of the antibody, or the like, and examples thereof may include Fab (Fragment of antigen binding), Fab', F(ab')₂, single chain Fv (scFv), disulfide stabilized Fv (dsFv), a fusion protein including an antibody Fc region and the like.

Examples of the antibody may also include antibodies prepared by the genetic recombination technique, or the like, that is, antibodies obtained by introduction of an antibody gene inserted antibody-expressing vector into a host cell, in addition to antibodies produced by hybridoma cells that are prepared from spleen cells of an immunized animal after immunization of the animal with an antigen. Specific examples thereof may include an antibody produced by hybridoma, human chimeric antibody, humanized antibody, human antibody and the like.

The human chimeric antibody means an antibody which is composed of heavy-chain variable region (hereinafter, heavy chain is referred to as H chain, and variable region is referred to as V region, namely, HV or VH) and light-chain variable region (hereinafter, light chain is referred to as L chain, namely, LV or VL) of an antibody of a non-human animal, and heavy chain constant region (hereinafter, constant region is referred to as C region, namely, CH) and light chain constant region (hereinafter, referred to as CL) of a human antibody. As the non-human animal, any animal, for example, mice, rats, hamsters, rabbits or the like, may be used as long as it can be used to produce hybridoma.

The human chimeric antibody may be prepared by obtaining cDNAs encoding VH and VL from a hybridoma capable of producing a monoclonal antibody, constructing a human chimeric antibody expression vector by inserting them into an expression vector for host cells having genes encoding human antibody CH and human antibody CL, and introducing them into host cells to express it.

The CH of the human chimeric antibody may be any one belonging to human immunoglobulins (hereinafter, referred to as hIg), and those of hIgG class are preferred. Further, any one of subclasses belonging to hIgG class such as hIgG1, hIgG2, hIgG3 or hIgG4 may be used. Further, the CL of the human chimeric antibody may be any one belonging to hIg, and those of κ class or λ class may be used.

Examples of the humanized antibody may include human type complementarity determining region (hereinafter, referred to as CDR)-grafted antibodies that are prepared by grafting an amino acid sequence of CDR of VH and VL of the antibody of the non-human animal to a proper region of VH and VL of the human antibody.

The CDR-grafted antibody may be prepared by constructing cDNAs encoding V regions in which CDR sequences of VH and VL of the antibody of the non-human animal have been grafted to CDR sequences of VH and VL of any human antibody, constructing a CDR-grafted antibody expression vector by inserting them into an expression vector for host cells having genes encoding human antibody CH and human antibody CL, and expressing the human CDR-grafted antibody by introducing the expression vector into the host cells.

The CH of the human CDR-grafted antibody may be any one belonging to hIg. Those of hIgG class are preferred. Further, any one of subclasses belonging to hIgG class such as hIgG1, hIgG2, hIgG3 or hIgG4 may be used. Further, the CL of the CDR-grafted antibody may be any one belonging to hIg, and those of κ class or λ class may be used.

With regard to the human antibody present in the human body, for example, a lymphocyte capable of producing the antibody may be cultured by isolating a human peripheral blood lymphocyte and infecting it with EB virus or the like to immortalize it, followed by cloning, and the antibody may be purified from the culture broth.

The human antibody phage library is a library in which antibody fragments such as Fab, scFv and the like are expressed on the phage surface by inserting an antibody gene prepared from human B cell into a phage gene.

From the library, a phage expressing an antibody fragment having an antigen binding activity can be recovered, using its activity to bind to a solid-phased antigen as the marker. The antibody fragment can be further converted into a human antibody composed of two complete H chains and two complete L chains.

The human antibody-producing transgenic animal is an animal in which a human antibody gene is introduced into cells. Specifically, the human antibody-producing transgenic mouse can be produced by introducing the human antibody gene into a mouse ES cell, transplanting the ES cell into an early stage embryo of a mouse and then generating it [Proc. Natl. Acad. Sci. USA, 97, 722(2000)].

The human antibody-producing hybridoma can be obtained from the human antibody-producing transgenic animal according to a hybridoma production method usually carried out in non-human mammals.

The human antibody can be produced by obtaining cDNAs encoding the VL and VH from the human antibody-producing hybridoma, inserting cDNAs into an expression vector for animal cells having DNAs encoding CL and CH of human antibody, respectively, and then introducing the expression vector into animal cells to express it.

The CH of WT used in the human antibody may be any one belonging to hIg. Those of hIgG class are preferred. Further, any one of subclasses belonging to hIgG class such as hIgG1, hIgG2, hIgG3 or hIgG4 may be used. Further, the CL of the human antibody may be any one belonging to hIg, and those of κ class or λ class may be used.

Specific examples of the antibodies prepared by the method of the present invention may include the following antibodies.

Examples of the antibodies recognizing tumor-associated antigens may include anti-GD2 antibody [Anticancer Res., 13, 331 (1993)], anti-GD3 antibody [Cancer Immunol. Immunother., 36, 260 (1993)], anti-GM2 antibody [Cancer Res., 54, 1511 (1994)], anti-HER2 antibody [Proc. Natl. Acad. Sci. USA, 89, 4285 (1992), US5725856], anti-CD52 antibody [Proc. Natl. Acad. Sci. USA, 89, 4285 (1992)], anti-MAGE antibody [British J. Cancer, 83, 493 (2000)], anti-HM 1.24 antibody [Molecular Immunol., 36, 387 (1999)], anti-parathyroid hormone related peptide (PTHrP) antibody [Cancer, 88, 2909 (2000)], anti-bFGF antibody, anti-FGF-8 antibody [Proc. Natl. Acad. Sci. USA, 86, 9911 (1989)], anti-bFGFR antibody, anti-FGF-8R antibody [J. Biol. Chem., 265, 16455 (1990)], anti-IGF antibody [J. Neurosci. Res., 40, 647 (1995)], anti-IGF-IR antibody [J. Neurosci. Res., 40, 647 (1995)], anti-PSMA antibody [J. Urology, 160, 2396 (1998)], anti-VEGF antibody [Cancer Res., 57, 4593 (1997)], anti-VEGFR antibody [Oncogene, 19, 2138 (2000), International Publication WO 96/30046], anti-CD20 antibody [Curr. Opin. Oncol., 10, 548 (1998), US Patent No. 5736137], anti-CD10 antibody, anti-EGFR antibody (International Publication WO 96/40201), anti-Apo-2R antibody (International Publication WO 98/51793), anti-ASCT2 antibody (International Publication WO 2010/008075), anti-CEA antibody [Cancer Res., 55 (23 suppl):5935s-5945s, (1995)], anti-CD38 antibody, anti-CD33 antibody, anti-CD22 antibody, anti-EpCAM antibody, anti-A33 antibody and the like.

Examples of the antibodies recognizing allergy or inflammation-associated antigens may include anti-interleukin 10 antibody [Immunol. Rev., 127, 5 (1992)], anti-interleukin 6 receptor antibody [Molecular Immunol., 31, 371 (1994)], anti-interleukin 5 antibody [Immunol. Rev., 127, 5 (1992)], anti-interleukin 5 receptor antibody, anti-interleukin 4 antibody [Cytokine, 3, 562 (1991)], anti-interleukin 4 receptor antibody [J. Immunol. Methods, 217, 41 (1998)], anti-tumor necrosis factor antibody [Lymph. Cyto. Res., 13, 183 (1994)], anti-tumor necrosis factor receptor antibody [Molecular Pharmacol., 58, 237 (2000)], anti-CCR4 antibody [Nature, 400, 776 (1999)], anti-chemokine antibody [Peri et al., J. Immunol. Meth., 174, 249 (1994)] or anti-chemokine receptor antibody [J. Exp. Med., 186, 1373 (1997)] and the like.

Examples of the antibodies recognizing cardiovascular disease-associated antigens may include anti-GPIIb/IIIa antibody [J. Immunol., 152, 2968 (1994)], antiplatelet-derived growth factor antibody [Science, 253, 1129 (1991)], anti-platelet-derived growth factor receptor antibody [J. Biol. Chem., 272, 17400 (1997)], anti-blood coagulation factor antibody [Circulation, 101, 1158 (2000)], anti-IgE antibody, anti-αVβ3 antibody, α4β7 antibody and the like.

Eamples of the antibodies recognizing viral or bacterial infection-associated antigens may include anti-gp120 antibody [Structure, 8, 385 (2000)], anti-CD4 antibody [J. Rheumatology, 25, 2065 (1998)], anti-CCR5 antibody, anti-verotoxin antibody [J. Clin. Microbiol., 37, 396 (1999)] and the like.

Examples of the peptide having a physiological activity may include a peptide retaining an activity of glycoprotein among the partial glycoprotein fragments, and the like. If the glycoprotein is an enzyme, a peptide regulating the activity of the enzyme or a peptide maintaining the structure of the enzyme is also included.

Specific example of the peptide regulating the activity of the enzyme may include peptides functioning as an agonist or an antagonist of the glycoprotein, or the like. Any agonist may be used as the agonist, as long as it is a peptide having an activity of accelerating the glycoprotein activity.

Specific example of the agonist may include somatostatin derivatives, somatropin, atrial natriuretic peptide, glucagon, insulin, insulin-like growth factor, gonadotropin and the like. Any antagonist may be used as the antagonist, as long as it is a peptide having an activity of inhibiting the glycoprotein activity, and specific example of the antagonist may include pegvisomant and the like.

The animal cells used in the production of the peptide having a physiological activity may be any animal cells, as long as they are able to produce the peptides. A transformant that is introduced with a vector having a gene encoding the peptide to be produced is preferably used.

The transformant cell that is introduced with a vector having a gene encoding the peptide may be obtained by introducing a recombinant vector including DNA encoding a peptide and a promoter into host cells.

The host cells may be animal cells having an ability to produce the above substances.

The vector used for the preparation of the recombinant vector may be any one of the vectors that function in the animal cells used in the present invention. Examples thereof may include pcDNAI, pcDM8 (manufactured by Funakoshi), pAGE106 [JP Patent Publication No. 3-22979], pAGE107 [Cytotechnology, 3, 133 (1990)], pAS3-3 (JP Patent Publication No. 2-227075), pcDM8 [Nature, 329, 840 (1987)], pcDNAI/Amp (manufactured by Invitrogen), pREP4 (manufactured by Invitrogen), pAGE103 [J. Biochem., 101, 1307 (1987), pAGE210 and the like.

The promoter may be any one of the promoters that function in the animal cells used in the present invention, and examples thereof may include a promoter of immediate early (IE) gene of cytomegalovirus (CMV), a SV40 early promoter, a retroviral promoter, a metallothionein promoter, a heat shock promoter, a SRα promoter and the like. An enhancer of IE gene of human CMV or the like may be also used, together with the promoter.

The introduction method of recombinant vector into host cells may be any method, as long as it is able to introduce DNA into the host cells, and examples thereof may include electroporation [Cytotechnology, 3, 133 (1990)], a calcium phosphate method [Virology, 52, 456 (1973)], lipofection [Proc. Natl. Acad. Sci. USA, 84, 7413 (1987)] and the like.

Specific examples of the transformed cells of the present invention may include anti-GD3 human chimeric antibody-producing transformed cell 7-9-51 (FERM BP-6691), anti-CCR4 chimeric antibody-producing transformed cell KM2760 (FERM BP-7054), anti-CCR4 humanized antibody-producing transformed cell KM8759 (FERM BP-8129) and KM8760 (FERM BP-8130), 709 LCA-500D (FERM BP-8239), anti-IL-5 receptor α chain chimeric antibody-producing transformed cell KM7399 (FERM BP-5649), anti-IL-5 receptor α chain human CDR-grafted antibody-producing transformed cell KM8399 (FERM BP-5648) and KM9399(FERM BP-5647), anti-GM2 human CDR-grafted antibody-producing transformed cell KM8966 (FERM BP-5105), KM8967 (FERM BP-5106), KM8969 (FERM BP-5527), KM8970(FERM BP-5528), anti-CD20 antibody-producing transformed cell line Ms704-CD20 (FERM BP-10092), antithrombin III-producing transformed cell Ms705-pKAN-ATIII (FERM BP-8472) and the like.

The method of culturing animal cells may be any one of batch culture, repeat batch culture, fed-batch (semi-batch, feeding) culture, perfusion culture and the like, as long as it is suitable for animal cells to be used. The fed-batch culture is preferably used.

Typically, for example, the fed-batch culture is carried out for 3 to 20 days and the perfusion culture is carried out for 3 to 60 days under the conditions of pH 6 to 8, 30 to 40°C or the like. If necessary, antibiotics such as gentamicin, streptomycin, penicillin or the like may be also added to the medium during culture. Meanwhile, control of dissolved oxygen concentration, pH, and temperature, stirring and the like may be carried out in accordance with the method generally used in the animal cell culture.

With respect to the culture method used in the present invention, the culture volume may be any one of low culture volume of 10 to 1000 mL using an Erlenmeyer flask or the like and high volume culture of 1 to 20000 L for commercial production using a culture tank or the like such as jar or the like.

Further, the present invention relates to a method for producing the substance by culturing animal cells having the ability to produce the substance, which is characterized by culturing the animal cells in a medium supplemented with an oligopeptide having one or more L-cysteines to produce and accumulate the substance in the culture, and collecting the substance from the culture.

The culture of adding an oligopeptide having one or more L-cysteines may be carried out by the above described method.

If the substance is a peptide, examples of the method for producing the substance of the present invention may include a direct expression method of producing the peptide inside the host cells, a method of producing the peptide by secreting the peptide outside the host cells (Molecular Cloning, second edition) or the like.

The peptide can be actively secreted outside the host cells by applying the method of Paulson, et al. [J. Biol. Chem., 264, 17619 (1989)], the method of Lowe, et al. [Proc. Natl. Acad. Sci. USA, 86, 8227 (1989), Genes Develop., 4, 1288 (1990)], or the methods described in JP Patent Publication No. 5-336963, International Publication WO 94/23021 or the like. That is, the desired peptide can be actively secreted outside the host cells by expressing the peptide in a form in which a signal peptide is added at the N-terminus of the desired peptide by using genetic engineering techniques.

It is also possible to increase the production level of the desired peptide by utilizing a gene amplification system which uses a dihydrofolate reductase gene or the like described in JP Patent Publication No. 2-227075.

The desired peptide produced by the above described method may be isolated and purified by the typical method of isolating and purifying the peptide, or the like.

When the desired peptide is expressed in a soluble form in the cells, after the completion of culture, the cells are recovered by centrifugation and suspended in an aqueous buffer, followed by disruption using a sonicator, a French press, a Manton Gaulin homogenizer, a Dynomill or the like to obtain a cell-free extract. A crude purified preparation or purified preparation can be obtained from the supernatant by centrifuging the cell-free extract using conventional methods for isolation and purification of peptides.

Examples of the method for isolation and purification of peptides may include solvent extraction, salting-out method with ammonium sulfate or the like, desalting, precipitation with an organic solvent, anion exchange chromatography using resins such as diethylaminoethyl-Sepharose, DIAION HPA-75 (manufactured by Mitsubishi Kasei) or the like, cation exchange chromatography using resins such as S-Sepharose FF (manufactured by Pharmacia) or the like, hydrophobic chromatography using resins such as butyl Sepharose, phenyl Sepharose or the like, gel filtration using a molecular sieve, affinity chromatography using resins containing protein A, protein G or the like, chromatofocusing, electrophoresis such as isoelectric focusing or the like, and the like. These methods may be used alone or in combination.

When the desired peptide is secreted outside the cells, the peptide can be recovered from the culture supernatant. That is, the culture is treated by centrifugation or the like in the same manner as above so as to obtain the culture supernatant. A crude purified preparation or purified preparation can be obtained from the culture supernatant by isolation and purification in the same manner as above.

### EXAMPLES

Hereinafter, the present invention will be described in more detail with reference to Examples. However, these Examples are for illustrative purposes only, and the invention is not intended to be limited by these Examples.

### [Example 1]

The following media for cell culture were prepared to compare differences in precipitation therebetween.

In 700 mL of water, 31.6 g of AGT Efficient Feed A (manufactured by Invitrogen, Catalog No. AG090231P7), 0.50 mL of polyamine solution (manufactured by Invitrogen, Catalog No. AG090232P8), 27.2 g of AGT Efficient Feed B (manufactured by Invitrogen, Catalog No. AG090230P7), 30.0 g of Hydrolysate (manufactured by DMV, Catalog No. P42474), 70.0 g of glucose (manufactured by Wako Pure Chemical Industries, Catalog No. 041-00595), and 5.0 g of L-(+)-glutamine (manufactured by Wako Pure Chemical Industries, Catalog No. 078-00525) or 7.4 g of L-alanyl-L-glutamine (manufactured by Kyowa Hakko Bio) were mixed.

After stirring for approximately 30 minutes, 7.0 mL of 5 mol/L HCl aqueous solution (manufactured by Wako Pure Chemical Industries, Catalog No. 081-05435) was added, followed by further stirring for approximately 20 minutes and pH was adjusted to 6.5. Thereafter, 50 mL of an alkaline solution containing 0.5 mol/L of NaOH (manufactured by Junsei Chemical, Catalog No. 39155-0301), 100 mmol/L of L-tyrosine disodium salt (manufactured by Sigma, Catalog No. T1145-100G), and 100 mmol/L of L-cystine dihydrochloride (manufactured by Wako Pure Chemical Industries, Catalog No. 034-05322) was added to the prepared aqueous solution of pH 6.5.

A volume of the aqueous solution was adjusted to 1 L with water, followed by stirring for approximately 20 minutes so as to prepare a medium for cell culture. In the medium for cell culture, L-tyrosine disodium salt and L-cystine dihydrochloride were substituted with the same concentration (5.0 mmol/L) of L-alanyl-L-tyrosine (manufactured by Kyowa Hakko Bio) and bis-L-alanyl-L-cysteine (manufactured by Kyowa Hakko Bio) to prepare media for cell culture, respectively. The prepared media for cell culture were stored under three conditions of at 4°C (refrigerator, manufactured by Hoshizaki), 20°C (e-Cooling Bucket, manufactured by Taitec), or room temperature.

As a result, precipitations were observed in the media containing L-tyrosine disodium salt and L-cystine dihydrochloride under all conditions, after 3 days. Among these, more precipitation was observed in the group stored at the low temperature of 4°C.

Meanwhile, there was no precipitation in the media for cell culture, which were prepared by substitution of L-tyrosine disodium salt and L-cystine dihydrochloride with L-alanyl-L-tyrosine (manufactured by Kyowa Hakko Bio) or bis-L-alanyl-L-cysteine (manufactured by Kyowa Hakko Bio), respectively.

These results indicate that precipitation in the medium can be inhibited by adding an oligopeptide having one or more L-cysteines and excluding glutathione to the medium for cell culture.

### [Example 2]

The following feed media for cell culture were prepared, and fed-batch culture was carried out using CHO cells for comparison.

The feed medium was composed of 31.6 g/L of AGT Efficient Feed A (manufactured by Invitrogen, Catalog No. AG090231P7), 0.50 ml/L of polyamine solution (manufactured by Invitrogen, Catalog No. AG090232P8), 27.2 g/L of AGT Efficient Feed B (manufactured by Invitrogen, Catalog No. AG090230P7), 5.0 g/L of L-(+)-glutamine (manufactured by Wako Pure Chemical Industries, Catalog No. 078-00525), 30.0 g/L ofhydrolysates (manufactured by DMV, Catalog No. P42474), 70.0 g/L of glucose (manufactured by Wako Pure Chemical Industries, Catalog No. 041-00595), 5.0 mmol/L of L-alanyl-L-tyrosine (Kyowa Hakko Bio), and 5.0 mmol/L, 10 mmol/L or 16 mmol/L of bis-L-alanyl-L-cysteine (manufactured by Kyowa Hakko Bio), and prepared in the same manner as in Example 1.

In the culture using antibody X-expressing CHO cells, 4.0 mmol/L of glutamine (manufactured by Invitrogen, Catalog No. 25030)-containing EX-CELL325 (manufactured by SAFC, Catalog No. 14340C-1000ML) was used as a main culture medium. The feed medium was added to perform fed-batch culture. The results are shown in Table 1.

**Table. 1**

| L-alanyl-L-cysteine disulfide (mmol/L) | Number of living cells (10⁵cells/mL) | Survival rate (%) |
|---|---|---|
| 5 | 28.6 | 55.0 |
| 10 | 44.1 | 78.7 |
| 16 | 58.1 | 86.6 |

As shown in Table 1, when the concentration of bis-L-alanyl-L-cysteine was changed from 5.0 mmol/L to 10 mmol/L or 16 mmol/L, it was observed that at 13 days of culture, the number of living cells was 28.6×10⁵ cells/mL and the survival rate was 55.0% at the concentration of 5.0 mmol/L, the number of living cells was 44.1 × 10⁵cells/mL and the survival rate was 78.7% at the concentration of 10 mmol/L, and the number of living cells was 58.1 × 10⁵cells/mL and the survival rate was 86.6% at the concentration of 16 mmol/L, and the number of living cells and the survival rate were increased in a bis-L-alanyl-L-cysteine concentration-dependent manner.

These results indicate that the number of living animal cells and the survival rate can be improved by culturing animal cells having an ability to produce a substance in the medium supplemented with an oligopeptide having one or more L-cysteines and excluding glutathione.

### [Example 3]

In order to examine antibody production, antibody titer was measured at 13 days of culture in the fed-batch culture of CHO cells having an ability to produce the antibody of Example 2. In the feed medium, the concentrations of bis-L-alanyl-L-cysteine were 0.75 g/L at 5.0 mmol/L, 0.79 g/L at 10 mmol/L, and 0.78 g/L at 16 mmol/L.

These results indicate that the substance can be produced from animal cells by culturing animal cells having an ability to produce a substance in the medium supplemented with an oligopeptide having one or more L-cysteines and excluding glutathione.

The present invention was described in detail with reference to specific embodiments. However, it will be apparent to those skilled in the art that various changes and modifications may be made without departing from the spirit and scope of the invention. Meanwhile, this application is based on Japanese Patent Application No. 2010-173546, filed on Aug. 2, 2010, and the disclosure thereof is incorporated herein by reference in its entirety.

### INDUSTRIAL APPLICABILITY

The present invention provides a method for culturing animal cells having an ability to produce a substance, which comprises culturing the animal cells in a medium supplemented with an oligopeptide having one or more L-cysteines and excluding glutathione, a method for producing a substance by culturing animal cells having the ability to produce the substance, which comprises culturing the animal cells in a medium supplemented with an oligopeptide having one or more L-cysteines and excluding glutathione to produce and accumulate the substance in the culture, and collecting the substance from the culture, and a culture medium comprising an oligopeptide having one or more L-cysteines and excluding glutathione.

## Claims

1. A method for culturing an animal cell having an ability to produce a substance, which comprises culturing the animal cell in a medium supplemented with an oligopeptide having one or more L-cysteines and excluding glutathione.

2. The method according to claim 1, wherein the oligopeptide having one or more L-cysteines is a dipeptide or a dimer of the dipeptide.

3. The method according to claim 2, wherein the dipeptide having one or more L-cysteines is glycyl-L-cysteine, L-alanyl-L-cysteine, or γ-glutamyl cysteine, or a salt thereof.

4. The method according to claim 2, wherein the dimer of the dipeptide having one or more L-cysteines is bis (glycyl-L-cysteine) or bis (L-alanyl-L-cysteine).

5. The method according to any one of claims 1 to 4, wherein the animal cell is cultured in a medium further supplemented with L-tyrosine or an oligopeptide having one or more L-tyrosines.

6. The method according to claim 5, wherein the oligopeptide having one or more L-tyrosines is a dipeptide.

7. The method according to claim 6, wherein the dipeptide having one or more L-tyrosines is L-alanyl-L-tyrosine or a salt thereof.

8. The method according to any one of claims 1 to 7, wherein the oligopeptide having one or more L-cysteines which is added to the medium has a concentration of from 0.01 to 100 mmol/L.

9. The method according to any one of claims 5 to 8, wherein L-tyrosine or the oligopeptide having one or more L-tyrosines which is added to the medium has a concentration of from 0.01 to 100 mmol/L.

10. The method according to any one of claims 1 to 9, wherein the animal cell is derived from an animal belonging to mammals.

11. The method according to claim 10, wherein the animal belonging to mammals belongs to primates or rodents.

12. The method according to claim 10 or 11, wherein the animal cell is a myeloma cell or an ovarian cell, or a cell derived therefrom.

13. The method according to any one of claims 1 to 12, wherein the substance is a peptide.

14. The method according to any one of claims 10 to 12, wherein the animal cell is a transformed cell to which a vector comprising a gene encoding the peptide is introduced.

15. The method according to claim 13 or 14, wherein the peptide is a glycoprotein.

16. The method according to claim 15, wherein the glycoprotein is an antibody.

17. The method according to any one of claims 1 to 16, wherein the oligopeptide is added at logarithmic growth phase of the cell.

18. The method according to any one of claims 1 to 17, wherein the medium supplemented with an oligopeptide is a feed medium.

19. The method according to any one of claims 1 to 18, wherein the culture method is a culture method using a culture tank.

20. A method for producing a substance by culturing an animal cell having an ability to produce a substance, which comprises culturing the animal cell in a medium supplemented with an oligopeptide having one or more L-cysteines and excluding glutathione to produce and accumulate the substance in the culture, and collecting the substance from the culture.

21. The method according to claim 20, wherein the oligopeptide having one or more L-cysteines is a dipeptide or a dimer of the dipeptide.

22. The method according to claim 21, wherein the dipeptide having one or more L-cysteines is glycyl-L-cysteine, L-alanyl-L-cysteine, or γ-glutamyl cysteine, or a salt thereof.

23. The method according to claim 21, wherein the dimer of the dipeptide having one or more L-cysteines is bis (glycyl-L-cysteine) or bis (L-alanyl-L-cysteine).

24. The method according to any one of claims 20 to 23, wherein the animal cell is cultured in a medium further supplemented with L-tyrosine or an oligopeptide having one or more L-tyrosines.

25. The method according to claim 24, wherein the oligopeptide having one or more L-tyrosines is a dipeptide.

26. The method according to claim 25, wherein the dipeptide having one or more L-tyrosines is L-alanyl-L-tyrosine or a salt thereof.

27. The method according to any one of claims 20 to 26, wherein the oligopeptide having one or more L-cysteines which is added to the medium has a concentration of from 0.01 to 100 mmol/L.

28. The method according to any one of claims 24 to 27, wherein L-tyrosine or the oligopeptide having one or more L-tyrosines which is added to the medium has a concentration of from 0.01 to 100 mmol/L.

29. The method according to any one of claims 20 to 28, wherein the animal cell is derived from an animal belonging to mammals.

30. The method according to claim 29, wherein the animal belonging to mammals belongs primates or rodents.

31. The method according to claim 29 or 30, wherein the animal cell is a myeloma cell or an ovarian cell, or a cell derived therefrom.

32. The method according to any one of claims 20 to 31, wherein the substance is a peptide.

33. The method according to any one of claims 29 to 31, wherein the animal cell is a transformed cell to which a vector comprising a gene encoding the peptide is introduced.

34. The method according to claim 32 or 33, wherein the peptide is a glycoprotein.

35. The method according to claim 34, wherein the glycoprotein is an antibody.

36. The method according to any one of claims 20 to 35, wherein the oligopeptide is added at logarithmic growth phase of the cell.

37. The method according to any one of claims 20 to 36, wherein the medium supplemented with the oligopeptide is a feed medium.

38. The method according to any one of claims 20 to 37, wherein the culture method is a culture method using a culture tank.

39. A medium comprising an oligopeptide having one or more L-cysteine and excluding glutathione.

40. The medium according to claim 39, wherein the oligopeptide having one or more L-cysteines and excluding glutathione is a dipeptide or a dimer of the dipeptide.

41. The medium according to claim 40, wherein the dipeptide having one or more L-cysteines is glycyl-L-cysteine, L-alanyl-L-cysteine, or γ-glutamyl cysteine, or a salt thereof.

42. The medium according to claim 41, wherein the dimer of the dipeptide having one or more L-cysteines is bis (glycyl-L-cysteine) or bis (L-alanyl-L-cysteine).

43. The medium according to any one of claims 39 to 42, wherein the medium further comprises L-tyrosine or an oligopeptide having one or more L-tyrosines.

44. The medium according to claim 43, wherein the oligopeptide having one or more L-tyrosines is a dipeptide.

45. The medium according to claim 44, wherein the dipeptide having one or more L-tyrosines is L-alanyl-L-tyrosine or a salt thereof.

46. The medium according to any one of claims 39 to 45, wherein the oligopeptide having one or more L-cysteines has a concentration of from 0.01 to 100 mmol/L.

47. The medium according to any one of claims 39 to 45, wherein a L-tyrosine or the oligopeptide having one or more L-tyrosines has a concentration of from 0.01 to 100 mmol/L.
